# FASCICULE DE BREVET EUROPEEN

(11) **EP 3 169 153 B1**
(45) Date de publication et mention de la délivrance du brevet: **03.04.2019**
(21) Numéro de dépôt: 15759848.3
(22) Date de dépôt: 17.07.2015
(51) Int. Cl.: A01N 1/02

(54) **EMBALLAGE DE MÉDICAMENT DE THÉRAPIE INNOVANTE**
VERPACKUNG FÜR EIN ARZNEIMITTEL ZUR VERWENDUNG IN EINER INNOVATIVEN THERAPIE
PACKAGING FOR A MEDICAMENT USED FOR INNOVATIVE THERAPY

(30) Priorité: 18.07.2014 FR 1456935
(43) Date de publication de la demande: 24.05.2017
(73) Titulaire: Laboratoire Français du Fractionnement et des Biotechnologies, 91940 Les Ulis (FR)
(72) Inventeur: HERNANDEZ, Benoît, 75010 Paris (FR); PRINGUET, Sébastien, F-91180 Saint Germain les Arpajon (FR)
(74) Mandataire: Cabinet Plasseraud
(86) Numéro de dépôt international: PCT/FR2015/051977
(87) Numéro de publication internationale: WO 2016/009162

(56) Documents cités:
- EP-A1- 2 668 847
- WO-A2-03/030769
- DE-U1-202010 011 159
- US-A1- 2014 021 208
- US-A1- 2014 157 799
- Anonymous: "Product Catalogue, Sofrigam", , 2 décembre 2012 (2012-12-02), XP055182461, Extrait de l'Internet: URL:http://web.archive.org/web/20121202064 415/http://www.sofrigam.com/media/pdf/cata log.pdf [extrait le 2015-04-13]
- World Health Organization: "Aide-memoire for prevention of freeze damage to vaccines", , 1 July 2009 (2009-07-01), XP055278057, Retrieved from the Internet: URL:http://apps.who.int/iris/bitstream/106 65/69673/1/WHO_IVB_07.09_eng.pdf [retrieved on 2016-06-06]
- Francesca Mercati ET AL: "Evaluation of storage conditions on equine adipose tissue-derived multipotent mesenchymal stromal cells", VETERINARY JOURNAL, vol. 200, no. 2, 1 May 2014 (2014-05-01), pages 339-342, XP055278060, GB ISSN: 1090-0233, DOI: 10.1016/j.tvjl.2014.02.018
- V. Antonenas ET AL: "Fresh PBSC harvests, but not BM, show temperature-related loss of CD34 viability during storage and transport", Cytotherapy, vol. 8, no. 2, 1 January 2006 (2006-01-01) , pages 158-165, XP055162427, ISSN: 1465-3249, DOI: 10.1080/14653240600620994
- N. Dib: "Safety and Feasibility of Autologous Myoblast Transplantation in Patients With Ischemic Cardiomyopathy: Four-Year Follow-Up", Circulation, vol. 112, no. 12, 20 September 2005 (2005-09-20), pages 1748-1755, XP055097125, ISSN: 0009-7322, DOI: 10.1161/CIRCULATIONAHA.105.547810

## Description

La présente invention se rapporte à un produit selon la revendication 1 et un procédé de transport selon la revendication 7.

Les substances biologiques, en particulier les médicaments de thérapie innovante, sont généralement constituées de matériaux vivants ou issus du vivant, tels que des acides nucléiques, des cellules isolées ou des tissus isolés. De telles substances sont relativement fragiles, et nécessitent des conditions de conservation et de stockage particulières pour maintenir leur activité.

L'utilité de ces substances est souvent entravée par des problèmes logistiques et notamment de conditions de transport. Par exemple, les cellules souches, telles que les cellules de sang de cordon, une fois recueillies, sont couramment cryoconservées, notamment dans des banques de cellules, et, en cas de besoin, peuvent être transportées jusqu'aux hôpitaux. Ce processus de cryoconservation, selon lequel les cellules ou les tissus sont conservés par refroidissement à des températures typiquement d'environ -196°C, comporte certains risques. Par exemple, les cellules peuvent être endommagées par le gel ou pendant le réchauffement à température ambiante.

Ces risques sont particulièrement graves pour le maintien de l'activité de la substance biologique, car ladite substance doit conserver son intégrité. En outre, les cellules et les tissus isolés utilisés doivent être viables pour être efficaces.

Le transport de ces substances biologiques doit donc s'effectuer sur une période de temps aussi courte que possible. En effet, les livraisons durant la nuit sur de la carboglace ou sous azote liquide constituent la norme, et des soins supplémentaires doivent être pris pour contrôler les températures tout au long du trajet. Pourtant, cette pratique n'élimine pas les risques. En outre, le transport utilisé est souvent coûteux et peu pratique sur une longue distance.

Le transport des vaccins, des cellules souches mésenchymateuses isolées, des cellules souches hématopoïétiques isolées et des myoblastes à une température réfrigérée est connu des documents "Aide-memoire for prevention of freeze damage to vaccines", World Health Organization, juillet 2009 ; Francesca Mercati et al., "Evaluation of storage conditions on equine adipose tissue-derived multipotent mesenchymal stromal cells", Veterinary Journal, vol. 200, no. 2, 2014, pages 339-342 ; V. Antonenas et al., "Fresh PBSC harvests, but not BM, show temperature-related loss of CD34 viability during storage and transport", Cytotherapy, vol. 8, no. 2, 2006, pages 158-165 ; et N. Dib et al., "Safety and Feasibility of Autologous Myoblast Transplantation in Patients With Ischemic Cardiomyopathy: Four-Year Follow-Up", Circulation, vol. 112, no. 12, 2005, pages 1748-1755. WO 03/030769 A2 divulgue un dispositif pour le stockage et transport de semences animales, comprenant un insert en mousse plastique avec des trous pour le stockage des seringues contenant les échantillons de semences, ledit insert étant placé dans un récipient isolant avec une poche de glace.

Il existe donc un besoin de disposer de produits et de procédés pour le transport de substances biologiques, qui soient pratiques, sûrs et qui assurent un maintien de l'intégrité et de la viabilité des substances biologiques concernées.
La présente invention permet de répondre à ce besoin. En effet, le produit selon l'invention, et le procédé de transport utilisant ce produit, assurent un suivi, et de bonnes conditions de conservation des substances biologiques au cours du temps. En effet, comme démontré dans les exemples, le transport peut être effectué pendant une durée de 48h, tout en maintenant la température fixée, i.e. 2-8°C ou 34-38°C.

La présente invention se rapporte donc à un produit selon la revendication 1 et à un procédé de transport selon la revendication 7.

Le produit et le procédé de transport selon la présente divulgation concernent des substances biologiques, que l'on peut qualifier de biopsie ou de médicaments de thérapie innovante.

Ces substances sont notamment choisies parmi :
- les médicaments de thérapie génique ;
- les médicaments de thérapie cellulaire somatique ;
- les médicaments issus de l'ingénierie cellulaire ou tissulaire ; et
- les médicaments combinés de thérapie innovante.
Les médicaments de thérapie génique sont généralement constitués d'acides nucléiques recombinants incorporés dans des outils adaptés (de type vecteurs, plasmides, particules virales...).
Les autres médicaments précités sont typiquement constitués de cellules ou de tissus isolés. Selon l'invention, la substance est choisie parmi les acides nucléiques recombinants, les myoblastes, les cellules souches mésenchymateuses isolées, les cellules souches hématopoïétiques isolées et les cellules immunocompétentes.
Les cellules souches hématopoïétiques peuvent être utilisées de façon allogénique ou autologue.
Les cellules immunocompétentes sont notamment choisies parmi les lymphocytes T allogéniques, les cellules NK, les lymphocytes T cytotoxiques, les lymphocytes infiltrant les tumeurs (TILs) et les cellules dendritiques sensibilisées avec un antigène, notamment tumoral.
Certains types cellulaires peuvent également être utilisés selon des exemples ne faisant pas partie de l'invention, comme les neurones foetaux dans les cas de maladies neurodégénératives, les îlots de Langerhans ou les hépatocytes en remplacement de greffes d'organes, ou bien les fibroblastes, les kératinocytes, les chondrocytes ou les cellules endothéliales dans les cas de médecine réparatrice.

Le produit selon l'invention comprend ainsi au moins une substance a) choisie parmi les acides nucléiques recombinants, les myoblastes, les cellules souches mésenchymateuses isolées, les cellules souches hématopoïétiques isolées et les cellules immunocompétentes. Cette substance, appelée également « substance biologique » dans la présente invention, est contenue dans un récipient primaire. Le récipient primaire est, quant à lui, contenu dans le récipient secondaire b), ledit récipient secondaire étant constitué d'un matériau isolant.

De préférence, le matériau isolant constituant le récipient secondaire est un polymère, de préférence un polymère plastique. De préférence, le polymère est choisi parmi le polypropylène, le polytetrafluoroéthylène (PTFE), le polyéthylène, le polycarbonate, le polyéthylènetéréphtalate (PETG), le chlorure de polyvinyle (PVC), le polyfluorure de vinylidène et leurs mélanges. Plus préférentiellement, le matériau isolant constituant le récipient secondaire est le polypropylène.
De préférence, le récipient secondaire est une boîte ou une caisse.
De préférence, le récipient primaire est choisi parmi une poche, un tube, un flacon, une ampoule, une seringue, un microtube et une plaque à puits. C'est lui qui est au contact de la substance.
La substance biologique peut être formulée dans un milieu biologiquement acceptable au sein du récipient primaire. Par « milieu biologiquement acceptable », on entend un milieu compatible avec ladite substance. Un tel milieu est notamment un milieu de culture cellulaire, par exemple comprenant du sérum comme du sérum humain.

Selon l'invention, le récipient secondaire est contenu dans un récipient tertiaire comprenant une unité de régulation thermique. Ledit récipient tertiaire est de préférence constitué d'une unité de régulation thermique, et de plaques d'isolation thermique. Selon des exemples qui ne font pas partie de l'invention, le récipient secondaire peut être également contenu dans n'importe quel autre type d'emballage tertiaire, par exemple un emballage en polystyrène ou un emballage de type panneau isolant sous vide (Vacuum Insulating Panel (VIP)).
De préférence, le produit selon l'invention comprend une sonde thermique, de préférence une sonde thermique filaire. Cette sonde est de préférence dans le récipient secondaire au contact du récipient primaire. Une telle sonde permet de suivre la température de la substance biologique au plus près, sans ouvrir les récipients concernés.

De préférence, le produit selon l'invention est revêtu d'une couche de matériau résistant aux rayons X, de préférence une feuille de plomb. Un tel matériau résistant aux rayons X permet de protéger la substance biologique, notamment lors des transports par avion.

De préférence, les récipients primaire, secondaire, et tertiaire, du produit selon l'invention, sont étanches. Ils assurent ainsi, chacun, l'isolement de la substance biologique, et notamment son transport de façon fiable.

L'invention a également pour objet un procédé de transport selon la revendication 7.
Le procédé de transport selon l'invention permet d'optimiser la manipulation, le maintien en température et de préserver l'intégrité du produit (donc de la substance biologique) vis-à-vis d'agressions extérieures pouvant être causées par les moyens réfrigérants ou chauffants.
Le transport du produit selon l'invention est possible avec un maintien de température pendant 72h.
De préférence, le procédé permet le transport de la substance de façon stable pendant une durée d'au moins 24h, de préférence pendant au moins 48h.
Par transport « de façon stable », on entend que le transport s'effectue sans détérioration substantielle de la substance biologique, ladite substance biologique conservant son intégrité physique et sa fonctionnalité. « Sans détérioration substantielle » signifie une destruction de la substance d'au plus 5% en poids.

L'invention va maintenant être exemplifiée à l'aide des exemples qui suivent, qui ne sont pas limitatifs.

### Exemple 1: transport de vaccin

Une substance biologique, en l'occurrence un vaccin, est transportée dans différentes conditions.
Le maintien de la température est évalué pendant la durée du transport.

Déterminer le temps de descente en température d'un produit stabilisé à température ambiante et introduit dans un caisson équipé d'une unité régulatrice de température, le tout placé dans une enceinte sous le profil ST96A

### 1-1 Récipient tertiaire (caisse)

Emballage: Caisson R11
Dimensions extérieures: 394 x 310 x 521 mm
Dimensions utiles: 171 x 267 x 224 mm
Isolant: Mousse de polyuréthane et panneaux de VIP
Séparations: une séparation en fibre plastique

### 1-2 Unité régulatrice de température

Désignation: Rigid Snowgam 10/05
Disposition: individuelle
Nombre de plaques: 1
Dimensions: 180 x 145 x 35 mm
Poids de la plaque : 670 g

Désignation: Rigid Snowgam 12/05
Disposition: individuelles
Nombre de plaques: 2
Dimensions: 230 x 180 x 35 mm
Poids de la plaque : 890 g

Désignation: TRU (Thermal Regulating Unit)
Disposition: individuel
Nombre: 1
Dimensions: 300 x 200 x 150 mm
Poids : 7.38 Kg

### 2- Mesure de la température

Thermoboutons utilisés et positions:
Thermobouton numéro: THB 03 Emplacement: Température ambiante
Thermobouton numéro: THB 08 Emplacement: Produit
Thermobouton numéro: THB 10 Emplacement: Intérieur caisson

### 3- Conditions d'essai

Type de produits: Seringue (récipient primaire) vide placée dans un emballage carton de dimensions 43 x 23 x 133 mm.

La seringue est placée à l'intérieur d'une boite en polypropylène de dimensions 200 x 190 x 110 mm (récipient secondaire). Du papier Kraft a été placé autour du vaccin pour le caler.

### 4- Conditions de chargement

Température : +21 (±3°C)
Disposition de la plaque Rigid Snowgam 10/05: au fond à +5°C
Disposition des plaques Rigid Snowgam 12/05: 1 sur chaque grand côté à +5°C
Disposition du TRU: sur le dessus à -21°C
Disposition de la séparation en fibre plastique : Entre le TRU et la boite polypropylène contenant le produit.

Le caisson R11 a été chargé avec les différents accumulateurs de froid ainsi que le TRU et la boite en polypropylène (avec calage) ; le tout placé dans l'enceinte de test durant 24 heures (24 premières heures du profil). Après ces 24 heures, le caisson a été ouvert et le vaccin (stabilisé à température ambiante) ajouté au chargement.

### Profil ST96A :

### ST96A

| Température | Durée |
|---|---|
| +22°C | 3H |
| +28°C | 4H |
| +22°C | 9H |
| +28°C | 8H |
| +40°C | 4H |
| +28°C | 3H |
| +22°C | 9H |
| +25°C | 8H |
| +22°C | 3H |
| +28°C | 4H |
| +22°C | 9H |
| +28°C | 8H |
| +40°C | 4H |
| +28°C | 3H |
| +22°C | 9H |
| +25°C | 8H |

### CONCLUSION

Dans cette configuration, la température d'un vaccin introduit à température ambiante dans le caisson équipé de 1 Rigid Snowgam 10/05, 2 Rigid Snowgam 12/05 et 1 TRU atteint les 8°C après 30 minutes de test.

Ensuite, le caisson est capable de maintenir le vaccin entre +2°C et +8°C pendant au moins 96 heures sous cette configuration.

### Exemple 2: Test de validation des propriétés isolantes du récipient secondaire à vide

Le but est de démontrer qu'à très basse température, l'emballage polypropylène (i.e. récipient secondaire) permet de réduire le choc thermique qui pourrait potentiellement altérer l'intégrité d'une substance choisie parmi les acides nucléiques recombinants, des cellules isolées et des tissus isolés.
L'autre objectif est de démontrer qu'après le transport, lorsque l'emballage secondaire en polypropylène se retrouve à température ambiante, ce dernier retarde la remontée en température et permet donc une manipulation plus sécurisante pour la substance.

Le test est le suivant :
Un emballage polypropylène est placé dans un congélateur <-60°C pendant 10h. Une sonde A (sonde de type Sensitech Temptale 4 Dry Ice permettant des mesures de +60°C à -110°C) est placée à l'intérieur de l'emballage, une autre B (du même type) à l'extérieur de l'emballage.
Ce test est effectué dans une salle à température régulée à 23°C.

Le test a commencé le 17/04/14 à 18h51 et s'est terminé le 18/04/14 à 10h07.

### Résultat :

### La sonde A était placée à l'intérieur de l'emballage :

### Descente en température :

Valeur maximale : 22,5 °C à 17/04/2014 18:51
Valeur congélation cible : <-60,0 °C à 17/04/2014 18:59
Temps de descente entre la valeur maximale (température ambiante) et la valeur congélation cible (température ambiante du congélateur) : **8 minutes**

### Remontée en température :

Valeur minimale : -77,5 °C à 18/04/2014 09:51
Valeur au-dessus de 0°C (2.6°C) à 18/04/2014 09:57
Valeur maximale : 22,9 °C à 18/04/2014 10:07
Temps de remontée entre la valeur minimale (température ambiante du congélateur) et la valeur maximale (température ambiante) : **16 minutes**
Temps de remontée entre la valeur minimale (température ambiante du congélateur) et la première valeur au-dessus de 0°C : **6 minutes**

### La sonde B était placée à l'extérieur de l'emballage :

### Descente en température :

Valeur maximale : 22,5 °C à 17/04/2014 18:51
Valeur congélation cible : <-60,0 °C à 17/04/2014 18:55
Temps de descente entre la valeur maximale (température ambiante) et la valeur congélation cible (température ambiante du congélateur) : **4 minutes**

### Remontée en température :

Valeur minimale : -77,9 °C à 18/04/2014 09:51
Valeur au-dessus de 0°C (10.6°C) à 18/04/2014 09:54
Valeur maximale : 22.7 °C à 18/04/2014 10:00
Temps de remontée entre la valeur minimale (température ambiante du congélateur) et la valeur maximale (température ambiante) **9 minutes**
Temps de remontée entre la valeur minimale (température ambiante du congélateur) et la première valeur au-dessus de 0°C : **3 minutes.**

### Conclusion :

On constate que l'emballage en polypropylène (récipient secondaire) assure une descente en température plus douce ainsi qu'une remontée en température plus lente. En effet l'ensemble des paramètres est en moyenne multiplié par deux.

### Exemple 3: Test de validation des propriétés isolantes du récipient secondaire

Deux flacons Duran Schott de 50ml remplis de 25ml d'eau chacun sont placés dans une chambre froide afin de prendre la température.
Le premier flacon est ensuite placé dans une sacoche de transport isotherme et entouré avec deux gels packs préalablement congelés à -80°C.

Le second flacon est tout d'abord placé dans l'emballage en polypropylène (récipient secondaire) avant d'être placé dans une sacoche de transport isotherme et entouré avec deux gels packs préalablement congelés à -80°C.
Chaque sacoche isotherme est équipée d'une sonde filaire, dont l'une sera placée à l'intérieur de l'emballage isotherme. Les sondes utilisées sont des sondes de type Sensitech Temptale 4 Dry Ice permettant des mesures de +60°C à -110°C. L'emballage isotherme est préalablement placé dans la chambre froide afin d'être également à température.

Le but est de démontrer que l'emballage polypropylène permet de réduire l'impact de la surcongélation causée par des gels packs trop congelés placés dans un camion frigorifique, ce qui pourrait potentiellement altérer l'intégrité d'une substance choisie parmi les acides nucléiques recombinants, des cellules isolées et des tissus isolés, ou encore fausser le relevé de température durant le transport.

### Résultat :

Le test a commencé le 22/04/14 à 16h36 et s'est terminé le 23/04/14 à 9h33.

On observe une chute de la température beaucoup moins importante mesurée par la sonde à l'intérieur de l'emballage en polypropylène, comparativement à température mesurée par la sonde placée directement au milieu des deux gels packs.

L'écart de chute de température est d'environ -20°C.

Ce test permet de conclure qu'un emballage en polypropylène placé entre deux gels packs préalablement congelés à -80°C pendant 10h et laissés à décongeler pendant environ 4h à l'intérieur d'un sac de transport isotherme, permet d'assurer un maintien de la température entre +2°C et +8°C pendant environ 13h.

### Conclusion :

On constate que l'emballage en polypropylène assure une chute en température beaucoup plus faible que le même produit non conditionné dans un emballage en polypropylène.

### Exemple 4: Test de validation des propriétés isolantes du récipient secondaire

Ce protocole vise à tester le déroulement et les conditions dans lesquels sont transportés les prélèvements. Une solution tampon ou de l'eau sont utilisés, pour simuler les tissus ou cellules prélevés.

L'objectif de ce rapport est de décrire le transfert de tissus/cellules, entre un hôpital et le site de production du médicament de thérapie innovante. Dans le but de mimer ce transfert de tissus/cellules prélevés, deux tests ont été réalisés :
- l'un en utilisant un tampon de formulation PBS, le 6 mai 2014, sur une température de +34/+38°C ;
- l'autre en utilisant de l'eau, le 17 septembre 2014, sur une température de +34/+38°C.

L'objectif est, à chaque fois, de garantir l'intégrité du flacon durant le transport, de l'enlèvement jusqu'à la livraison, à température contrôlée.

### Matériel utilisé :

### Tampon ou eau :

Le tampon de formulation mimant le prélèvement (premier test) a été préparé au préalable sur le site hospitalier. Un tube Sarstedt de 50ml contenant ce tampon, a été ainsi préparé et placé dans un incubateur à +37°C le 06/05/14 à 10h00.

| | Tube Sarstedt |
|---|---|
| Nom du produit | PBS |
| Description | Tampon de formulation |
| Dimension | d.2cm h.10cm |
| Volume | 50ml |
| Etiquetage | NA |

L'eau mimant le prélèvement (second test) a été préparée au préalable sur le site hostpialier. Un tube Sarstedt de 50ml contenant cette eau, a été ainsi préparée et placée dans un incubateur à +37°C du CHU de Nantes le 17/09/14 à 10h00.

| | Tube Sarstedt |
|---|---|
| Description | Eau |
| Dimension | d.2cm h.10cm |
| Volume | 50ml |
| Etiquetage | NA |

### Emballage extérieur :

L'emballage extérieur se compose d'un caisson avec des plaques.

Cet emballage a été préparé pour maintenir une température de +34/+38°C.

### Protocole d'emballage :

Le tube Sarstedt contenant le PBS ou l'eau est placé dans une pochette plastique 95 Kpa 11 de contenant (emballage secondaire), puis dans une boîte en polypropylène (200x190x110mm) puis dans l'emballage extérieur (394 x 310 x 521 mm).

### Sondes :

Deux sondes ont été utilisées pour ce transport test :
- une sonde intégrée à l'emballage, programmée à +34/+38°C ;
- une sonde Thermobouton programmée avec un intervalle de mesures de 3 min d'enregistrement. Température +34/+38°C.

Les résultats du premier test sont les suivants :

| **Actions** | **Temps écoulé** | **Date** | **Heure** | **Température interne [+34°C ; +38°C]** |
|---|---|---|---|---|
| **Démarrage sondes** | 00:00:00 | *05*/*05*/*14* | 09:00:00 | 23,2°C |
| **Mise en place des accumulateurs** | 01:51:00 | *05*/*05*/*14* | 10:51:00 | 21,9°C |
| **Montée en température** | 02:06:00 | *05*/*05*/*14* | 11:06:00 | 34,1°C |
| **Départ emballage** | 02:51:00 | *05*/*05*/*14* | 11:51:00 | 37,6°C |
| **Ouverture : Manipulation au point A** | 05:24:00 | *05*/*05*/*14* | 14:24:00 | 36,2°C |
| **Arrivée emballage sur le site hospitalier** | 1 jour 04:15:00 | *06*/*05*/*14* | 13:15:00 | 35,5°C |
| **Ouverture : Ajout du prélèvement** | 1 Jour 04:27:00 | *06*/*05*/*14* | 13:27:00 | 35,1°C |
| **Départ emballage + prélèvement** | 1 Jour 04:36:00 | *06*/*05*/*14* | 13:36:00 | 34,5°C |
| **Arrivée emballage + prélèvement au point A** | 2 Jours 03:15:00 | *07*/*05*/*14* | 12:15:00 | 35,4°C |
| **Ouverture : Récupération du prélèvement** | 2 Jours 05:45:00 | *07*/*05*/*14* | 14:45:00 | 35,4°C |
| **Température < à 34°C** | 5 Jours 14:33:00 | *10*/*05*/*14* | 23:33:00 | 33,9°C |

| **Index** | ***Température Interne (C°)*** |
|---|---|
| **Spécification** | [+34,0 ; +38,0] °C |
| **Maximum** | 37,8°C |
| **Minimum** | 21,9°C |
| **Moyenne durant le transport** *[05*/*05*/*2014 10:51 - 07*/*05*/*2014 14:45]* | 35,5°C |

Le tube est arrivé dans les bonnes conditions, et respectant les consignes de températures durant le transport.

Les résultats du second test sont les suivants :

| **Actions** | **Temps écoulé** | **Date** | **Heure** | **Température interne [+34°C ; +38°C]** |
|---|---|---|---|---|
| **Démarrage sondes** | 00:00:00 | *16*/*09*/*14* | 11 :18 | 36,1°C |
| **Départ emballage : Sofrigam vers Ulis** | 00:06:00 | *16*/*09*/*14* | 11 :24 | 36,3°C |
| **Ouverture : Manipulation CfC Ulis** | 03:27:00 | *16*/*09*/*14* | 14 :45 | 35,5°C |
| **Arrivée emballage sur le site du CHU de Nantes** | 21 :24:00 | *17*/*09*/*14* | 08 :42 | 35,5°C |
| **Ouverture : Ajout prélèvement CHU de Nantes** | 1 Jour 00:33:00 | *17*/*09*/*14* | 11 :51 | 34,8°C |
| **Départ emballage + prélèvement CHU de Nantes** | 1 Jour 00:36:00 | *17*/*09*/*14* | 11 :54 | 34,8°C |
| **Arrivée emballage + prélèvement CfC Ulis** | 2 Jours 01:18:00 | *18*/*09*/*14* | 12 :36 | 35,4°C |
| **Ouverture** : **Récupération prélèvement CfC Ulis** | 2 Jours 04:54:00 | *18*/*09*/*14* | 16 :18 | 30,1°C |
| **Remontée en température après fermeture** | 2 Jours 05:15:00 | *18*/*09*/*14* | 16 :33 | 34,2°C |
| **Température < à 34°C** | 6 Jours 02:15:00 | *22*/*09*/*14* | 13 :33 | 32,5°C |

| **Index** | ***Température Interne (C°)*** |
|---|---|
| **Spécification** | [+34,0 ; +38,0] °C |
| **Maximum** | 37,8°C |
| **Minimum** | 24.9°C |
| **Moyenne durant le transport** *[17*/*09*/*2014 11:51 - 18*/*09*/*2014 16:18]* | 35,5°C |

Le tube est arrivé dans les bonnes conditions, et respectant les consignes de températures durant le transport.

En conclusion ces transports sont conformes : la matière transportée a été maintenue à bonne température, et ce pendant au moins 48h.

## Revendications

1. Produit comprenant :
a) au moins une substance choisie parmi les acides nucléiques recombinants, les myoblastes, les cellules souches mésenchymateuses isolées, les cellules souches hématopoïétiques isolées et les cellules immunocompétentes, ladite substance étant contenue dans un récipient primaire ; et
b) un récipient secondaire, dans lequel est contenu le récipient primaire, ledit récipient secondaire étant constitué d'un matériau isolant ; et
c) un récipient tertiaire comprenant une unité de régulation thermique ;
et ledit récipient secondaire étant contenu dans le récipient tertiaire.

2. Produit selon la revendication 1, **caractérisé en ce que** le matériau isolant constituant le récipient secondaire est un polymère choisi parmi le polypropylène, le polytetrafluoroéthylène, le polyéthylène, le polycarbonate, le polyéthylènetéréphtalate, le chlorure de polyvinyle, le polyfluorore de vinylidène et leurs mélanges,

3. Produit selon l'une des revendications 1 à 2, **caractérisé en ce que** le récipient primaire est choisi parmi une poche, un tube, un flacon, une ampoule, une seringue, un microtube et une plaque à puits.

4. Produit selon l'une des revendications 1 à 3, **caractérisé en ce qu'**il comprend une sonde thermique, de préférence une sonde thermique filaire.

5. Produit selon l'une des revendications 1 à 4, **caractérisé en ce qu'**il est revêtu d'une couche de matériau résistant aux rayons X, de préférence une feuille de plomb.

6. Produit selon l'une des revendications 1 à 5, **caractérisé en ce que** les récipients primaire, secondaire, et tertiaire, sont étanches.

7. Procédé de transport d'une substance choisie parmi les acides nucléiques recombinants, les myoblastes, les cellules souches mésenchymateuses isolées, les cellules souches hématopoïétiques isolées et les cellules immunocompétentes, comprenant les étapes suivantes **:**
i) placer ladite substance dans un récipient primaire ;
ii) placer ledit récipient primaire obtenu à l'étape i) dans un récipient secondaire, ledit récipient secondaire étant constitué d'un matériau isolant;
iii) fermer le récipient secondaire obtenu à l'étape ii) ;
placer ledit récipient secondaire dans un récipient tertiaire, ledit récipient tertiaire comprenant une unité de régulation thermique, et ainsi obtenir un produit selon l'une des revendications 1 à 6.

8. Procédé selon la revendication 7 pour préserver l'intégrité de la substance vis-à-vis d'agressions extérieures.

## Patentansprüche

1. Produkt, umfassend:
a) wenigstens eine Substanz, welche gewählt ist aus rekombinanten Nukleinsäuren, Myoblasten, isolierten mesenchymalen Stammzellen, isolierten hämatopoetischen Stammzellen und immunkompetenten Zellen, wobei die Substanz in einem Primärbehälter enthalten ist; und
b) einen Sekundärbehälter, in welchem der Primärbehälter enthalten ist, wobei der Sekundärbehälter aus einem Isoliermaterial besteht; und
c) einen Tertiärbehälter, welcher eine thermische Regeleinheit umfasst; und wobei der Sekundärbehälter in dem Tertiärbehälter enthalten ist.

2. Produkt nach Anspruch 1, **dadurch gekennzeichnet, dass** das den zweiten Behälter bildende Isoliermaterial ein Polymer ist, welches gewählt ist aus Polypropylen, Polytetrafluorethylen, Polyethylen, Polycarbonat, Polyethylenterephthalat, Polyvinylchlorid, Polyfluorid, Vinylidenpolyfluorid und deren Mischungen.

3. Produkt nach einem der Ansprüche 1 bis 2,
**dadurch gekennzeichnet, dass** der Primärbehälter gewählt ist aus einer Tasche, einem Röhrchen, einem Flakon, einer Ampulle, einer Spritze, einem Mikroröhrchen und einer Platte mit Vertiefungen.

4. Produkt nach einem der Ansprüche 1 bis 3,
**dadurch gekennzeichnet, dass** es einen Temperaturfühler, vorzugsweise einen Drahttemperaturfühler umfasst.

5. Produkt nach einem der Ansprüche 1 bis 4,
**dadurch gekennzeichnet, dass** es mit einer Schicht aus röntgenstrahlenresistentem Material bedeckt ist, vorzugsweise mit einer Bleifolie.

6. Produkt nach einem der Ansprüche 1 bis 5,
**dadurch gekennzeichnet, dass** die Primär-, Sekundär- und Tertiärbehälter abgedichtet sind.

7. Transportverfahren für eine Substanz, welche gewählt ist aus rekombinanten Nukleinsäuren, Myoblasten, isolierten mesenchymalen Stammzellen, isolierten hämatopoetischen Stammzellen und immunkompetenten Zellen, umfassend die folgenden Schritte:
i) Platzieren der Substanz in einem Primärbehälter;
ii) Platzieren des in Schritt i) erhaltenen Primärbehälters in einem Sekundärbehälter, wobei der Sekundärbehälter aus Isoliermaterial besteht;
iii) Verschließen des in Schritt ii) erhaltenen Sekundärbehälters;
Platzieren des Sekundärbehälters in einem Tertiärbehälter, wobei der Tertiärbehälter eine thermische Regeleinheit umfasst, und derart Erhalten eine Produkts nach einem der Ansprüche 1 bis 6.

8. Verfahren nach Anspruch 7 zum Erhalt der Integrität der Substanz gegenüber Umwelteinflüssen.

## Claims

1. Product comprising:
a) at least one substance selected from among recombinant nucleic acids, myoblasts, isolated mesenchymal stem cells, isolated hematopoietic stem cells and immunocompetent cells, said substance being contained in a primary container; and
b) a secondary container, wherein is contained the primary container, said secondary container being constituted of an insulating material; and
c) a tertiary container comprising a thermal regulation unit;
and said secondary container being contained in the tertiary container.

2. Product according to claim 1, **characterised in that** the insulating material constituting the secondary container is a polymer selected from among polypropylene, polytetrafluoroethylene, polyethylene, polycarbonate, polyethylene terephthalate, polyvinyl chloride, polyvinylidene fluoride and the mixtures thereof.

3. Product according to one of claims 1 to 2, **characterised in that** the primary container is selected from among a pocket, a tube, a vial, a bulb, a syringe, a microtube and a well plate.

4. Product according to one of claims 1 to 3, **characterised in that** it comprises a thermal probe, preferably a wired thermal probe.

5. Product according to one of claims 1 to 4, **characterised in that** it is coated with a layer of material which is resistant to X-rays, preferably a lead sheet.

6. Product according to one of claims 1 to 5, **characterised in that** the primary, secondary and tertiary containers are leaktight.

7. Method for transporting a substance selected from among recombinant nucleic acids, myoblasts, isolated mesenchymal stem cells, isolated hematopoietic stem cells and immunocompetent cells, comprising the following steps:
i) placing said substance in a primary container;
ii) placing said primary container obtained in step i) in a secondary container, said secondary container being constituted of an insulating material;
iii) closing the secondary container obtained in step ii);
placing said secondary container in a tertiary container, said tertiary container comprising a thermal regulation unit, and thus obtaining a product according to one of claims 1 to 6.

8. Method according to claim 7 to preserve the integrity of the substance facing external aggressors.
